# EUROPEAN PATENT APPLICATION

(11) **EP 2 455 070 A1**
(43) Date of publication of application: **23.05.2012**
(21) Application number: 12155487.7
(22) Date of filing: 29.12.2008
(51) Int. Cl.: A61K 9/48, A61K 31/232, A61K 31/366, A61K 31/40, A61P 3/06, A61P 43/00

(54) **Capsule Formulation**

(30) Priority: 10.01.2008 JP 2008003634
(62) Divisional of application: 08870164.4
(71) Applicant: Takeda Pharmaceutical Company Limited, Osaka-shi, Osaka 541-0045 (JP)
(72) Inventor: Uchiyama, Yoshihiro, Osaka, 532-8686 (JP); Yoshinari, Tomohiro, Osaka, 540-8645 (JP)
(74) Representative: Dossmann, Gérard

(57) **Abstract**

The present invention provides the following capsule preparation, which is superior in dissolution property and the like of a pharmaceutically active ingredient and contains liquid and solid pharmaceutically active ingredients: a seamless capsule containing liquid and solid pharmaceutically active ingredients, wherein the liquid pharmaceutically active ingredient is encapsulated in the form of a liquid pharmaceutical composition, and the solid pharmaceutically active ingredient is dispersed in a capsule shell layer.

## Description

### Technical Field

The present invention relates to a seamless capsule comprising one or more kinds of liquid pharmaceutically active ingredients and one or more kinds of solid pharmaceutically active ingredients.

### (Background of the Invention)

In recent years, single pharmaceutical preparations containing plural pharmaceutically active ingredients (combination agent) have been actively developed in the field of pharmaceutical products in an attempt to achieve synergistic action of efficacy, reduction of side effects, improved convenience for patients and the like.
Regarding a preparation with a liquid pharmaceutically active ingredient, patent document 1 describes a preparation containing only one pharmaceutically active ingredient (single agent), wherein a ω3-alkyl ester is filled in a soft gelatin capsule.
Regarding seamless capsules, patent documents 2 and 3 describe production methods thereof.
patent document 1: JP-B-2810916
patent document 2: JP-A-10-506841
patent document 3: JP-B-3159724

### Disclosure of the Invention

### Problems to be Solved by the Invention

In a combination agent, each of the plural pharmaceutically active ingredients contained therein needs to show bioabsorbability equivalent to that of a single agent containing the corresponding pharmaceutically active ingredient alone. In a combination agent, moreover, since the dissolution rates of plural pharmaceutically active ingredients from the combination agent can influence the time-course efficacy profile after administration of the combination agent, the composition and constitution need to be adjusted when designing the combination agent, such that the dissolution rate of each of the plural pharmaceutically active ingredients contained therein is optimized. In a combination agent, furthermore, each pharmaceutically active ingredient also needs to be present stably. However, adjustment of dissolution property and stability of each pharmaceutically active ingredient in a combination agent is not easy because plural pharmaceutically active ingredients contained therein show different properties. Not limited to combination agents, solid preparations are required to also permit easy portability and easy administration. However, combination agents generally have larger size as compared to single agents. Particularly, a combination agent containing a liquid pharmaceutically active ingredient and a solid pharmaceutically active ingredient, which is satisfactory in portability and easy administration, has not been developed. Furthermore, since combination agents generally require complicated production steps, production costs tend to become high.

### Means of Solving the Problems

The present inventors have conducted intensive studies in an attempt to solve the aforementioned problems and found that the above-mentioned problems can be solved by a seamless capsule comprising one or more kinds of liquid pharmaceutically active ingredients and one or more kinds of solid pharmaceutically active ingredients, particularly, a seamless capsule having the following characteristics:
(A) a liquid pharmaceutically active ingredient is encapsulated in the form of a liquid pharmaceutical composition, and a solid pharmaceutically active ingredient is dispersed in a capsule shell layer (to be referred to as "seamless capsule (A)" in the present specification);
(B) a liquid pharmaceutically active ingredient is encapsulated in the form of a liquid pharmaceutical composition, and a solid pharmaceutically active ingredient is dispersed in the liquid pharmaceutical composition (to be referred to as "seamless capsule (B)" in the present specification); or
(C) a liquid pharmaceutically active ingredient is encapsulated in the form of a liquid pharmaceutical composition, and a solid pharmaceutically active ingredient is applied onto a capsule shell layer free of the pharmaceutically active ingredient (to be referred to as "seamless capsule (C)" in the present specification); and conducted further studies, which resulted in the completion of the present invention.

Accordingly, the present invention relates to the following:
[1] a seamless capsule comprising one or more kinds of liquid pharmaceutically active ingredients and one or more kinds of solid pharmaceutically active ingredients;
[2] the seamless capsule of the above-mentioned [1], which is seamless capsule (A);
[3] the seamless capsule of the above-mentioned [2], further comprising a shell layer free of a pharmaceutically active ingredient on the outside of the capsule shell layer comprising a solid pharmaceutically active ingredient dispersed therein;
[4] the seamless capsule of the above-mentioned [1], which is seamless capsule (B);
[5] the seamless capsule of the above-mentioned [1], which is seamless capsule (C);
[6] the seamless capsule of any of the above-mentioned [1] to [5], wherein the liquid pharmaceutically active ingredient is a ω3-fatty acid ethyl ester;
[7] the seamless capsule of any of the above-mentioned [1] to [5], wherein the liquid pharmaceutical composition comprises not less than 90% w/w of a ω3-fatty acid ethyl ester;
[8] the seamless capsule of the above-mentioned [7], wherein the ω3-fatty acid ethyl ester comprises EPA ethyl ester and DHA ethyl ester and the liquid pharmaceutical composition comprises not less than 80% w/w in total of these two components;
[8a] the seamless capsule of the above-mentioned [7], wherein the ω3-fatty acid ethyl ester comprises EPA ethyl ester and DHA ethyl ester and the liquid pharmaceutical composition comprises not less than 70% w/w in total of these two components;
[9] the seamless capsule of the above-mentioned [8], wherein the liquid pharmaceutical composition comprises not less than 40% w/w of EPA ethyl ester and not less than 34% w/w of DHA ethyl ester;
[10] the seamless capsule of the above-mentioned [9], wherein the liquid pharmaceutical composition is OMEGA-3-ACID ETHYL ESTERS 90 in the European Pharmacopoeia;
[11] the seamless capsule of any of the above-mentioned [1] to [5], wherein the solid pharmaceutically active ingredient is lapaquistat acetate;
[12] the seamless capsule of any of the above-mentioned [1] to [5], wherein the solid pharmaceutically active ingredient is statin;
[13] the seamless capsule of the above-mentioned [12], wherein the solid pharmaceutically active ingredient is atorvastatin;
[14] the seamless capsule of the above-mentioned [12], wherein the solid pharmaceutically active ingredient is simvastatin.

### Effect of the Invention

The seamless capsule of the present invention has advantages in that (1) each of the liquid and solid pharmaceutically active ingredients can achieve bioabsorbability equivalent to that of a corresponding single agent; (2) the dissolution property of each of the liquid and solid pharmaceutically active ingredients can be controlled; (3) each active ingredient can be present stably; (4) the preparation is superior in portability and easy administration since it can be down-sized; (5) production costs can be suppressed than conventional combination agents since production steps can be simplified; and the like.

### Brief Description of the Drawings

Fig. 1 is a graph showing the blood concentration profile of lapaquistat acetate. Since the plasma concentration at the time point of 48 hr was 0 for any preparation, it is not described in the graph.
Fig. 2 is a graph showing the blood concentration profile of simvastatin. Since the plasma concentration at the time point of 48 hr was 0 for any preparation, it is not described in the graph.

### (Detailed Description Of The Invention)

The present invention is explained in detail in the following.
In the present specification, the above-mentioned "seamless capsule comprising one or more kinds of liquid pharmaceutically active ingredients and one or more kinds of solid pharmaceutically active ingredients", as well as "seamless capsule (A)", "seamless capsule (B)" and "seamless capsule (C)" are sometimes collectively referred to as the seamless capsule of the present invention.

The seamless capsule of the present invention consists of a capsule content and a capsule shell layer.

### (capsule content)

The content to be sealed in the seamless capsule of the present invention is a liquid pharmaceutical composition. Here, the liquid pharmaceutical composition may comprise a liquid pharmaceutically active ingredient and an additive, or a liquid pharmaceutically active ingredient per se (without additive). In addition, the liquid pharmaceutically active ingredient contained in the liquid pharmaceutical composition may be a combination of two or more kinds of pharmaceutically active ingredients.

As the additive, those generally used in the field of pharmaceutical products are used. Examples of such additive include inactive diluents (e.g., medium-chain triglyceride (MCT) oil, olive oil, soybean oil, corn oil, water, ethanol, a mixture thereof); pH adjusters (e.g., citrate, phosphate, carbonate, tartrate, fumarate, acetate, amino acid salt); surfactants (e.g., sodium lauryl sulfate, polysorbate 80, polyoxyethylene(160)polyoxypropylene(30)glycol); stabilizers (e.g., tocopherol, sodium tetraedetate, amide nicotinic acid, cyclodextrins); acidulants (e.g., ascorbic acid, citric acid, tartaric acid, malic acid); flavors (e.g., menthol, peppermint oil, lemon oil, vanillin); fluidizers (e.g., light anhydrous silicic acid, hydrated silicon dioxide, talc); and the like.
The seamless capsule of the present invention is preferably free of both the "interfacial tension modifier" and the "gelling agent". Even when the above-mentioned additives include one having an action to change interfacial tension and/or an action to shorten the gelling time, it may be used for the seamless capsule of the present invention as long as the amount thereof is insufficient to significantly change the interfacial tension and insufficient to significantly shorten the gelling time.

While the liquid pharmaceutically active ingredient is not particularly limited as long as it is liquid at 5 - 60°C, the seamless capsule of the present invention is advantageous for the formulation of a compound having easily oxidizable properties and oily compound.
A preferable example of such liquid pharmaceutically active ingredient is ω3-fatty acid alkyl ester (e.g., preferably ω3-fatty acid C₁₋₆ alkyl (e.g., methyl, ethyl, propyl, isopropyl, butyl, isobutyl, sec-butyl, tert-butyl, pentyl, hexyl) ester; more preferably ω3-fatty acid ethyl ester) and the like useful for the treatment of hyperlipidemia (e.g., hypercholesterolemia, high-LDL cholesterolemia, low-HDL cholesterolemia, hypertriglyceride(TG)mia) and the like, prevention of cardiovascular event and the like. Here, examples of the "ω3-fatty acid" of ω3-fatty acid alkyl ester include C₁₈₋₂₂ ω3-fatty acid (e.g., octadecatrienoic acid, octadecatetraenoic acid, eicosatetraenoic acid, eicosapentaenoic acid, heneicosapentaenoic acid, docosapentaenoic acid, docosahexaenoic acid).
More specific examples of the ω3-fatty acid ethyl ester include octadecatrienoic acid ethyl ester, octadecatetraenoic acid ethyl ester, eicosatetraenoic acid ethyl ester, eicosapentaenoic acid (EPA) ethyl ester, heneicosapentaenoic acid ethyl ester, docosapentaenoic acid ethyl ester, docosahexaenoic acid (DHA) ethyl ester, and a mixture of two or more kinds thereof (e.g., OMEGA-3-ACID ETHYL ESTERS 90 in the European Pharmacopoeia).
When the liquid pharmaceutically active ingredient is a ω3-fatty acid alkyl ester, the liquid pharmaceutical composition preferably contains not less than 90% w/w (90 - 100% w/w) of the ω3-fatty acid ethyl ester.
The composition of the ω3-fatty acid ethyl ester preferably consists of EPA ethyl ester and DHA ethyl ester in total of not less than 70% w/w (70 - 100% w/w), preferably not less than 80% w/w (80 - 100% w/w), more preferably not less than 40% w/w (40 - 66% w/w) of EPA ethyl ester and not less than 34% w/w (34 - 60% w/w) of DHA ethyl ester, and not less than 80% w/w (80 - 100% w/w) in total.

Preferable specific examples of the liquid pharmaceutical composition to be used in the present invention are as follows:
1) a composition comprising ω3-fatty acid alkyl ester at not less than 90%w/w (e.g., 90 - 100%w/w), wherein EPA ethyl ester and/or DHA ethyl ester are/is the main component(s);
2) the composition of the above-mentioned 1) comprising both components of EPA ethyl ester and DHA ethyl ester, wherein the total content ratio of the both components is not less than 80%w/w (e.g., 80 - 100%w/w; preferably 80 - 90%w/w) (here, the composition may contain ω3-fatty acid alkyl ester other than the EPA ethyl ester and DHA ethyl ester);
3) the composition of the above-mentioned 2) comprising EPA ethyl ester in not less than 40%w/w (e.g., 40 - 66%w/w; preferably 40 - 50%w/w) and DHA ethyl ester in not less than 34%w/w (e.g., 34 - 60%w/w; preferably 34 - 45%w/w) wherein the total content ratio of the EPA ethyl ester and the DHA ethyl ester is not less than 80%w/w, and the composition may contain ω3-fatty acid alkyl ester other than the EPA ethyl ester and DHA ethyl ester;
4) the composition of the above-mentioned 3) which is OMEGA-3-ACID ETHYL ESTERS 90 defined by the European Pharmacopoeia.
   The above-mentioned liquid pharmaceutical compositions 1) - 4) may be constituted by ω3-fatty acid alkyl ester alone, or may further contain additive(s).
As such additive, those exemplified above can be mentioned. Of those, a stabilizer (preferably, α-tocopherol) is preferable. When a stabilizer is to be used, its content in the liquid pharmaceutical composition is 0.3 - 0.5%w/w.
In the above, %w/w is the content ratio to the total weight of the liquid pharmaceutical composition.

In the seamless capsule (B) of the present invention, the above-mentioned liquid pharmaceutical composition further comprises one or more kinds of the below-mentioned solid pharmaceutically active ingredients. Such liquid pharmaceutical composition can be prepared, for example, by dispersing a solid pharmaceutically active ingredient in a liquid pharmaceutical composition. In this case, the solid pharmaceutically active ingredient may be finally dissolved in the liquid pharmaceutical composition.

### (shell composition)

The capsule shell layer (film) covering the capsule content of the seamless capsule of the present invention is formed by a shell composition comprising gelatin and a plasticizer, which does not substantially comprise an interfacial tension modifier and a gelling agent.

As the "gelatin", one generally used for the production of a seamless capsule, such as the pharmaceutical gelatin defined in the Japanese Pharmacopoeia Fifteenth Edition can be mentioned. The gelatin here includes modified gelatin such as succinylated gelatin and the like. Gelatin preferable for practicing the present invention includes those having the following properties.
1) The jelly strength of gelatin (measured based on the method described in JIS) is 200 - 300 g, more preferably 240 - 280 g.
2) The viscosity of gelatin (measured based on the method described in JIS) is 2 - 6 mPa·s, more preferably 3 - 5 mPa·s.
3) The gelatin is derived from pigskin.
   Particularly, gelatin satisfying one or more of the above-mentioned properties is more preferable. Gelatin to be used may be a combination of two or more kinds.

As the "plasticizer", those generally used for the production of seamless capsules can be mentioned and, for example, polyvalent alcohols such as glycerin (e.g., concentrated glycerin), ethylene glycol, polyethylene glycol, propylene glycol, polypropylene glycol and the like, and sugar alcohols such as sorbitol and the like are preferable. These plasticizers may be used in a combination of two or more kinds thereof.
Among those, preferred are glycerin and sorbitol.
It is also preferable to use them in a mixture. In this case, the weight ratio of glycerin and sorbitol is preferably within the range of 1:5 - 5:1, more preferably 1:3 - 3:1.

In the seamless capsule of the present invention, the shell composition preferably comprises gelatin and a plasticizer at a weight ratio within the range of 10:1 - 1:10, more preferably 10:1 - 1:1.

The weight ratio of the capsule content (i.e., liquid pharmaceutical composition) to "gelatin and plasticizer in total" in the shell composition is generally 10:1 - 1:10, preferably 10:1 - 1:3.

The weight ratio of the shell composition and the capsule content (i.e., liquid pharmaceutical composition) is generally 10:1 - 1:10, preferably 3:1 - 1:10.

### (Interfacial tension modifier and gelling agent)

In the seamless capsule of the present invention, a shell composition is characteristically substantially free of both an "interfacial tension modifier" and a "gelling agent" conventionally widely used for the production of seamless capsules.
The capsule content does not comprise both an "interfacial tension modifier" and a "gelling agent" in the same manner.
Here, the "interfacial tension modifier" refers to a substance that changes the tension produced in the oil-water interface between the capsule content and the aqueous shell composition solution. Examples thereof include phospholipid, fats and oils, surfactant, polar organic solvents such as alcohol and the like, and the like.
The "gelling agent" refers to a substance that promotes gelling of an aqueous shell composition solution by cooling. Examples thereof include polar organic solvents such as alcohol and the like, polysaccharides and the like.
Being "substantially free of an interfacial tension modifier and a gelling agent" means
1) being completely free of both components in a seamless capsule, or
2) even when both components or either component are/is contained in a seamless capsule, the content thereof being "insufficient to significantly change the interfacial tension" or "insufficient to significantly shorten the gelling time".
   As used herein, the "amount to significantly change the interfacial tension" is, for example, an amount causing a change of not less than 5 mN/m, or an amount causing a change of not less than 10%, as compared to no addition of an "interfacial tension modifier", when a tension produced in the oil-water interface between an aqueous gelatin solution (21.25% w/w, 50°C) and a content liquid (24°C) is measured. The interfacial tension is measured by, for example, a pendant drop method using FTA200 of First Ten Angstroms, Inc.
   The "amount to significantly shorten the gelling time" means, for example, an amount that shortens the time necessary for gelling upon dropwise addition of an aqueous gelatin solution (21.25%w/w) heated to 55°C to MCT oil at 4°C, namely, the time necessary for reaching 50% level of elasticity of an aqueous gelatin solution at 4°C, by not less than 10%, as compared to no addition of a "gelling agent".

The seamless capsule of the present invention may contain components other than the "interfacial tension modifier" and "gelling agent" when desired and, for example, may contain a colorant. A colorant to be contained in the composition is not particularly limited and a desired colorant is used as necessary. For example, food colors such as yellow dye No. 5, red dye No. 2, blue dye No. 2 and the like; β carotene; food lake colors; red ferric oxide, yellow ferric oxide; and the like can be mentioned.

When desired, moreover, the capsule may contain components such as monosaccharides (e.g., pentose such as arabinose, xylose, ribose, 2-deoxyribose and the like; hexose such as glucose, fructose, galactose, mannose, sorbose, rhamnose, fucose and the like), disaccharides (e.g., malt sugar, cellobiose, α,α-trehalose, lactose, sucrose), celluloses (e.g., crystalline cellulose (including microcrystalline cellulose)), inorganic products (e.g., anhydrous calcium phosphate, precipitated calcium carbonate, calcium silicate) and the like.
When any of the above-mentioned components has an effect of changing the interfacial tension and/or an effect of shortening the gelling time, the seamless capsule of the present invention may even contain a component having such effect in an amount insufficient to significantly change the interfacial tension and insufficient to significantly shorten the gelling time.

In the seamless capsule (A) of the present invention, the above-mentioned shell composition further comprises one or more kinds of solid pharmaceutically active ingredients. Such shell composition can be prepared, for example, by dispersing a solid pharmaceutically active ingredient in an inert solvent (e.g., water, ethanol or a mixture thereof) to give a dispersion of the solid pharmaceutically active ingredient, and then mixing the dispersion of the solid pharmaceutically active ingredient and the below-mentioned aqueous shell composition solution.

Moreover, the seamless capsule (B) of the present invention may further comprise one or more kinds of solid pharmaceutically active ingredients in the above-mentioned shell composition.

In the present invention, the solid pharmaceutically active ingredient to be combined with the liquid pharmaceutically active ingredient is not particularly limited, and it is appropriately selected in consideration of the effect of combined use with the liquid pharmaceutically active ingredient (e.g., synergistic action of efficacy, reduction of side effects, improved convenience for patients etc.).
For example, when the liquid pharmaceutically active ingredient is the above-mentioned ω3-fatty acid alkyl ester, HMG-CoA reductase inhibitors (e.g., statins such as cerivastatin, pravastatin, simvastatin, lovastatin, atorvastatin, fluvastatin, itavastatin, rosuvastatin, pitavastatin and the like, or a salt thereof (e.g., sodium salt, calcium salt)); squalene synthase inhibitors (e.g., compounds described in WO97/10224, preferably, lapaquistat acetate); and the like are used in consideration of the effect of combined use as a therapeutic agent for hyperlipidemia. Among these, atorvastatin, simvastatin and lapaquistat acetate are preferable.

Alternatively, as a solid pharmaceutically active ingredient to be combined with a ω3-fatty acid alkyl ester, therapeutic agents for diabetes, therapeutic agents for diabetic complications, antihypertensive agents, antiobesity agents, diuretics, chemotherapeutic agents, immunotherapeutic agents, antithrombotic agents, therapeutic agents for osteoporosis, antidementia agents, agents for ameliorating erectile dysfunction, therapeutic agents for urinary incontinence or pollakiuria, therapeutic agents for dysuria and the like can be used.

As therapeutic agents for diabetes, insulin preparations (e.g., animal insulin preparations extracted from pancreas of bovine and swine; human insulin preparations genetically synthesized using *Escherichia coli,* yeast; zinc insulin; protamine zinc insulin; fragment or derivative of insulin (e.g., INS-1 etc.), oral insulin preparation and the like), insulin sensitizers (e.g., pioglitazone or a salt thereof (preferably hydrochloride), rosiglitazone or a salt thereof (preferably maleate), Tesaglitazar, Ragaglitazar, Muraglitazar, Edaglitazone, Metaglidasen, Naveglitazar, AMG-131, THR-0921), α-glucosidase inhibitors (e.g., voglibose, acarbose, miglitol, emiglitate), biguanides (e.g., metformin, buformin or a salt thereof (e.g., hydrochloride, fumarate, succinate)), insulin secretagogues [e.g., sulfonylurea (e.g., tolbutamide, glibenclamide, gliclazide, chlorpropamide, tolazamide, acetohexamide, glyclopyramide, glimepiride, glipizide, glybuzole), repaglinide, nateglinide, mitiglinide or calcium salt hydrate thereof], dipeptidyl peptidase IV inhibitors (e.g., Alogliptin, Vildagliptin, Sitagliptin, Saxagliptin, T-6666, TS-021), β3 agonists (e.g., AJ-9677), GPR40 agonists, GLP-1 receptor agonists [e.g., GLP-1, GLP-1MR agent, NN-2211, AC-2993 (exendin-4), BIM-51077, Aib(8,35)hGLP-1(7,37)NH₂, CJC-1131], amylin agonists (e.g., pramlintide), phosphotyrosine phosphatase inhibitors (e.g., sodium vanadate), gluconeogenesis inhibitors (e.g., glycogen phosphorylase inhibitors, glucose-6-phosphatase inhibitors, glucagon antagonists), SGLUT (sodium-glucose cotransporter) inhibitors (e.g., T-1095), 11β-hydroxysteroid dehydrogenase inhibitors (e.g., BVT-3498), adiponectin or agonist thereof, IKK inhibitors (e.g., AS-2868), leptin resistance improving drugs, somatostatin receptor agonists, glucokinase activators (e.g., Ro-28-1675), GIP (Glucose-dependent insulinotropic peptide) and the like can be mentioned.

As therapeutic agents for diabetic complications, aldose reductase inhibitors (e.g., tolrestat, epalrestat, zenarestat, zopolrestat, minalrestat, fidarestat, CT-112, ranirestat (AS-3201)), neurotrophic factor and increasing drugs thereof (e.g., NGF, NT-3, BDNF, neurotrophin production-secretion promoters described in WO01/14372 (e.g., 4-(4-chlorophenyl)-2-(2-methyl-1-imidazolyl)-5-[3-(2-methylphenoxy)propyl]oxazole)), PKC inhibitors (e.g., ruboxistaurin mesylate), AGE inhibitors (e.g., ALT946, pimagedine, N-phenacylthiazolium bromide (ALT766), EXO-226, Pyridorin, pyridoxamine), active oxygen scavengers (e.g., thioctic acid), cerebral vasodilators (e.g., tiapuride, mexiletine), somatostatin receptor agonists (e.g., BIM23190), and apoptosis signal regulating kinase-1 (ASK-1) inhibitors can be mentioned.

As antihypertensive agents, angiotensin converting enzyme inhibitors (e.g., captopril, enalapril, delapril), angiotensin II antagonists (e.g., candesartan cilexetil, losartan, eprosartan, valsartan, telmisartan, irbesartan, olmesartan medoxomil, tasosartan, 1-[[2'-(2,5-dihydro-5-oxo-4H-1,2,4-oxadiazol-3-yl)biphenyl-4-yl]methyl]-2-ethoxy-1H-benzoimidazole-7-carboxylic acid), calcium channel blockers (e.g., manidipine, nifedipine, nicardipine, amlodipine, efonidipine), potassium channel openers (e.g., levcromakalim, L-27152, AL0671, NIP-121), clonidine and the like can be mentioned.

As antiobesity agents, for example, antiobesity agents acting on the central nervous system (e.g., dexfenfluramine, fenfluramine, phentermine, sibutramine, amfepramone, dexamphetamine, mazindol, phenylpropanolamine, clobenzorex; MCH receptor antagonists (e.g., SB-568849; SNAP-7941; compounds described in WO01/82925 and WO01/87834); neuropeptide Y antagonists (e.g., CP-422935); cannabinoid receptor antagonists (e.g., SR-141716, SR-147778); ghrelin antagonists; 11β-hydroxysteroid dehydrogenase inhibitors (e.g., BVT-3498)), pancreatic lipase inhibitors (e.g., orlistat, cetilistat (ATL-962)), β3 agonists (e.g., AJ-9677), peptide anorexiants (e.g., leptin, CNTF (Ciliary Neurotropic Factor)), cholecystokinin agonists (e.g., lintitript, FPL-15849), feeding deterrents (e.g., P-57) and the like can be mentioned.

As diuretics, for example, xanthine derivatives (e.g., sodium salicylate and theobromine, calcium salicylate and theobromine), thiazide preparations (e.g., ethiazide, cyclopenthiazide, trichloromethiazide, hydrochlorothiazide, hydroflumethiazide, benzylhydrochlorothiazide, penflutizide, polythiazide, methyclothiazide), antialdosterone preparations (e.g., spironolactone, triamterene), carbonate dehydratase inhibitors (e.g., acetazolamide), chlorobenzenesulfonamide preparations (e.g., chlortalidone, mefruside, indapamide), azosemide, isosorbide, etacrynic acid, piretanide, bumetanide, furosemide and the like can be mentioned.

As chemotherapeutic agents, for example, alkylating agents (e.g., cyclophosphamide, ifosfamide), metabolic antagonists (e.g., methotrexate, 5-fluorouracil and a derivative thereof (e.g., furtulon, Neo-Furtulon)), antitumor antibiotics (e.g., mitomycin, adriamycin), plant-derived antitumor agents (e.g., vincristine, vindesine, taxol), cisplatin, carboplatin, etoposide and the like can be mentioned.

As immunotherapeutic agents, for example, microorganism or bacterial components (e.g., muramyl dipeptide derivatives, Picibanil), polysaccharides with immunity enhancing activity (e.g., lentinan, schizophyllan, krestin), cytokines obtained by genetic engineering techniques (e.g., interferon, interleukin (IL) (e.g., IL-1, IL-2, IL-12)), colony stimulating factors (e.g., granulocyte colony stimulating factor, erythropoietin) and the like can be mentioned.

As antithrombotic agents, for example, heparins (e.g., heparin sodium, heparin calcium, dalteparin sodium), warfarins (e.g., warfarin potassium), anti-thrombin drugs (e.g., aragatroban), thrombolytic agents (e.g., urokinase, tisokinase, alteplase, nateplase, monteplase, pamiteplase), platelet aggregation inhibitors (e.g., ticlopidine hydrochloride, cilostazol, ethyl icosapentate, beraprost sodium, sarpogrelate hydrochloride) and the like can be mentioned.

As therapeutic agents for osteoporosis, for example, alfacalcidol, calcitriol, elcatonin, calcitonin salmon, estriol, ipriflavone, risedronate disodium, pamidronate disodium, alendronate sodium hydrate, incadronate disodium and the like can be mentioned.

As antidementia agents, for example, tacrine, donepezil, rivastigmine, galanthamine and the like can be mentioned.

As agents for ameliorating erectile dysfunction, for example, apomorphine, sildenafil citrate and the like can be mentioned.

As therapeutic agents for urinary incontinence or pollakiuria, for example, flavoxate hydrochloride, oxybutynin hydrochloride, propiverine hydrochloride and the like can be mentioned.

As therapeutic agents for dysuria, for example, acetylcholine esterase inhibitors (e.g., distigmine) and the like can be mentioned.

The seamless capsule of the present invention may use one or more kinds of solid pharmaceutically active ingredients.

Each component of the shell composition is, for example, blended and dissolved in an aqueous medium (e.g., water, ethanol and mixture thereof) and used for the production of a seamless capsule (the obtained solution is referred to as an "aqueous shell composition solution").
The concentration of the components (component concentration) other than water in the "aqueous shell composition solution" is preferably 15 - 35% w/w. When a low viscosity gelatin is used, the component concentration can be increased, which in turn shortens the drying time after production of a seamless capsule. Here, the low viscosity means 2 - 4 mPa·s. The viscosity is measured, for example, according to the method described in JIS.

### (Carrier liquid)

For production of the seamless capsule of the present invention, a "carrier liquid" generally used in this field can be employed.
Examples of the "carrier liquid" generally used in this field include oily substrates such as medium-chain triglyceride (MCT) oil, olive oil, soybean oil, corn oil and the like.

### (Preparation of seamless capsule)

The seamless capsule of the present invention is prepared using the above-mentioned "aqueous shell composition solution" and "capsule content" as materials, and a capsule production apparatus generally used for the production of seamless capsules (e.g., "SPHEREX" manufactured by Freund Corporation) and by a dropping immersed in a carrier liquid using multiple nozzles for the both components. To be more specific, a "capsule content" flows out from an inner nozzle of multiple nozzles into a carrier liquid and an "aqueous shell composition solution" from an outer nozzle thereof into the carrier liquid at constant rates, a fluid of these two layers is cut at given intervals to form droplets with an interfacial tension, and the outer shell layer is gelled by cooling to give seamless capsules. The obtained seamless capsules are cooled for a given time and dried to yield products.
However, production of the seamless capsule of the present invention is not limited to such production method and can also be produced by other method known in this field.

The shape of the seamless capsule of the present invention is round, teardrop and the like, by which the present invention is not limited.
The size of the seamless capsule of the present invention can be appropriately adjusted by a known method. A capsule having a diameter within the range of 1 - 10 mm is preferable, and a capsule having a diameter within the range of 2 - 6 mm is more preferable.

For the production of the seamless capsule of the present invention, it is preferable that the temperature conditions at the capsule formation point (near multiple nozzles) be appropriately controlled during production.
When the main component of a capsule content shows an excessively low or excessively high interfacial tension at a temperature near 25°C, as compared to appropriate interfacial tension, (especially when the content of the main component showing an excessively low or excessively high interfacial tension in the capsule content is high), it is particularly preferable for the production of a high quality seamless capsule to appropriately control the temperature.
In the present invention, the appropriate interfacial tension is, for example, 15 - 50 mN/m in the interface with an aqueous gelatin solution (21.25% w/w, 50°C). An interfacial tension below this level can be classified as being "excessively low", and an interfacial tension above this level can be classified as being "excessively high". The interfacial tension can be measured, for example, by the aforementioned method. This numerical value is one index that varies within a reasonable range depending on the kind of the capsule content to which the present invention is applied. Those of ordinary skill in the art can consider the necessity of temperature control as appropriate at specific situations where the present invention is applied.

The seamless capsule of the present invention may further have a shell layer free of a pharmaceutically active ingredient on the outside of a capsule shell layer comprising a solid pharmaceutically active ingredient dispersed therein. As such shell layer, the below-mentioned coating is used.

### (coating)

In one embodiment, the seamless capsule of the present invention may be further coated from the aspects of easy administration, increased strength of the preparation, control of dissolution property of the pharmaceutically active ingredient from the preparation and the like. The coating layer to be formed on the outside of a capsule shell layer here may or may not contain a pharmaceutically active ingredient.

In the seamless capsule (C) of the present invention, the solid pharmaceutically active ingredient is contained in a coating layer rather than a shell layer. That is, seamless capsule (C) has a coating layer comprising one or more kinds of solid pharmaceutically active ingredients on the outside of a capsule shell layer free of the solid pharmaceutically active ingredient.
Seamless capsule (C) can be produced by the following steps:
1) in the same manner as in the above-mentioned steps, a seamless capsule (capsule containing liquid pharmaceutically active ingredient) having a shell layer free of a solid pharmaceutically active ingredient is prepared;
2) a solid pharmaceutically active ingredient and a coating base and, where necessary, a coating additive are dispersed in or mixed with an inert solvent (e.g., water, ethanol or a mixture thereof) to give a solid pharmaceutically active ingredient dispersion coating solution (wherein the solid pharmaceutically active ingredient may be dissolved in the coating solution);
3) the coating solution obtained in the above-mentioned 2) is sprayed on the capsule obtained in the above-mentioned 1) by a rotary fluidized-bed granulator (e.g., MP-10; POWREX CORPORATION) and dried.

In the seamless capsules (A) and (B) of the present invention, the coating layer preferably does not contain a pharmaceutically active ingredient.
When a coating layer free of a pharmaceutically active ingredient is formed on seamless capsules (A) and (B), the above-mentioned production method of seamless capsule (C) excluding the solid pharmaceutically active ingredient can be employed.

Preferable examples of the coating base include sugar coating base, water-soluble film coating base, enteric film coating base, sustained-release film coating base and the like.
As sugar coating base, saccharides and sugar alcohols such as sucrose (including white soft sugar, granulated sugar), mannitol, erythritol and the like are used, and one or more kinds selected from talc, crystalline cellulose (including microcrystalline cellulose), precipitated calcium carbonate, gelatin, gum arabic (including gum arabic powder), hydroxypropylmethylcellulose, titanium oxide, pullulan, carnauba wax and the like may be used in combination.

As water-soluble film coating base, for example, cellulose polymers such as hydroxypropylcellulose, hydroxypropylmethylcellulose (e.g., hypromellose 2910), hydroxyethylcellulose, methylhydroxyethylcellulose and the like; synthetic polymers such as polyvinylacetal diethylaminoacetate, aminoalkyl methacrylate copolymer E [Eudragit E (trade name)], polyvinylpyrrolidone and the like; polysaccharides such as pullulan, sodium alginate and the like; and the like can be mentioned.

As enteric film coating base, for example, cellulose polymers such as hydroxypropylmethylcellulose phthalate, hydroxypropylmethylcellulose acetate succinate, carboxymethylethylcellulose, cellulose acetate phthalate and the like; acrylic acid polymers such as methacrylic acid copolymer L [Eudragit L (trade name)], methacrylic acid copolymer LD [Eudragit L-30D55 (trade name)], methacrylic acid copolymer S [Eudragit S (trade name)] and the like; natural products such as shellac and the like; and the like can be mentioned.

As sustained-release film coating base, for example, cellulose polymers such as ethylcellulose and the like; acrylic acid polymers such as aminoalkyl methacrylate copolymer RS [Eudragit RS (trade name)], ethyl acrylate-methyl methacrylate copolymer suspension [Eudragit NE (trade name)] and the like; and the like can be mentioned.

Preferable examples of coating additive include light shielding agents such as titanium oxide and the like, colorants such as red ferric oxide, yellow ferric oxide and the like; plasticizers such as polyethylene glycol (e.g., macrogol 6000), triethyl citrate, castor oil, polysorbates and the like; organic acids such as citric acid, tartaric acid, malic acid, ascorbic acid and the like; and the like.
The above-mentioned additives may be used in a mixture of two or more kinds thereof at an appropriately ratio.

For the production of the seamless capsule of the present invention, operations such as dispersion, stirring, coating and the like are performed according to methods conventionally used in the technical field of pharmaceutical preparations.
Dispersion can be performed, for example, by using a dispersion apparatus and the like.
Stirring can be performed, for example, by using a stirring device such as propeller stirrer, stir bar and the like.
Coating can be performed, for example, by using a fluidized granulator, a film coating apparatus and the like.

When a shell composition contains a solid pharmaceutically active ingredient, an inactive intermediate layer may be formed between the capsule content and the capsule shell layer to avoid interaction caused by the contact of a liquid pharmaceutically active ingredient in the capsule and a solid pharmaceutically active ingredient in the capsule shell layer.
The intermediate layer can be formed by molding, simultaneously with a shell layer containing a pharmaceutically active ingredient, the aforementioned shell composition free of a pharmaceutically active ingredient, a coating base and/or a coating additive as materials by using multiple nozzles during capsule formation.

The component ratio of the capsule shell layer to the whole preparation may be changed for the purpose of controlling the dissolution property of the pharmaceutically active ingredient from the preparation.

The component ratio of the coating layer (shell layer) free of a pharmaceutically active ingredient, which is formed on the outside of the capsule shell layer, to the whole preparation may be changed for the purpose of controlling the dissolution property of the pharmaceutically active ingredient from the preparation.

The seamless capsule of the present invention may be filled in a different capsule (e.g., hard capsule).
The seamless capsule of the present invention and a capsule filled with the seamless capsule may have a mark or letters for identification.

The capsule preparation of the present invention can be safely administered to mammals (e.g., mouse, rat, rabbit, cat, dog, bovine, horse, monkey, human).

The dose of the seamless capsule of the present invention need only be an effective amount of each pharmaceutically active ingredient to be contained in the seamless capsule. While the administration frequency of the seamless capsule of the present invention to the aforementioned mammals in one day varies depending on the properties of the pharmaceutically active ingredient to be contained, it is typically 1 - 3 times a day.

As a specific example, the effective amount of OMEGA-3-ACID ETHYL ESTERS 90 is 100 - 6000 mg/day for an adult (body weight 60 kg).
An effective amount of lapaquistat acetate is, for example, 1 - 100 mg/day for an adult (body weight 60 kg).
An effective amount of statin is, for example, 1 - 100 mg/day for an adult (body weight 60 kg).

The number of seamless capsules to be ingested per administration is appropriately set according to the content of the pharmaceutically active ingredient in one capsule.
When plural seamless capsules are to be ingested per administration, seamless capsules in the number necessary for one administration may be filled in one or several packages or capsules (e.g., hard capsules).

Specific examples of a particularly preferable seamless capsule of the present invention include the following:
a seamless capsule (B) containing 25 mg of OMEGA-3-ACID ETHYL ESTERS 90 and 1.25 mg of lapaquistat acetate per capsule;
a seamless capsule (B) containing 25 mg of OMEGA-3-ACID ETHYL ESTERS 90 and 2 mg of simvastatin per capsule;
a seamless capsule (B) containing 25 mg of OMEGA-3-ACID ETHYL ESTERS 90 and 2 mg of atorvastatin per capsule;
a seamless capsule (A) containing 25 mg of OMEGA-3-ACID ETHYL ESTERS 90 and 1.25 mg of lapaquistat acetate per capsule;
a seamless capsule (A) containing 25 mg of OMEGA-3-ACID ETHYL ESTERS 90 and 2 mg of simvastatin per capsule;
a seamless capsule (C) containing 25 mg of OMEGA-3-ACID ETHYL ESTERS 90 and 1.25 mg of lapaquistat acetate per capsule; and
a seamless capsule (C) containing 25 mg of OMEGA-3-ACID ETHYL ESTERS 90 and 2 mg of simvastatin per capsule.

In view of the content uniformity and stability of each pharmaceutically active ingredient during long-term preservation, seamless capsules (A) and (C) are preferable.
In view of the simplification of production steps, seamless capsule (B) is preferable.

### Examples

The present invention is explained in more detail in the following Examples, Control Examples and Evaluation Examples, which are not to be construed as limitative.
As preparation additives, those listed in the Japanese Pharmacopoeia Fifteenth Edition, the Japanese Pharmacopoeia Pharmaceutical Codex or Pharmaceutical Excipients 2003, were used in the following Examples and Control Examples.
In the following Examples, the composition ratio shows weight ratio.

### Example 1

According to the composition ratio in the following Table 1, the seamless capsule (B) of the present invention was produced.
Gelatin (jelly strength 250 g) (2548.3 g), concentrated glycerin (300.7 g), sorbitol solution (214.8 g) (solid content: 150.36 g), and yellow dye No. 5 (0.63 g) were dissolved in purified water (6935.56 g) heated to 52°C, and the mixture was degassed under reduced pressure to give an aqueous shell composition solution.
To OMEGA-3-ACID ETHYL ESTERS 90 (6000 g) was added powdery lapaquistat acetate (300 g), and the solid pharmaceutically active ingredient was dispersed by using a dispersion apparatus (manufactured by POLYTRON, KINEMATICA AG) to give a capsule content.
Using an apparatus for seamless capsule production (SPHEREX, manufactured by Freund Corporation), a seamless capsule was prepared from the aqueous shell composition solution and the capsule content. Using MCT oil as a carrier liquid, capsulation was performed at 25 capsules per second.
The obtained capsules were cooled in a refrigerator, and dried in a drier until the water activity reached not more than 0.2. Then the carrier liquid on the capsule surface was wiped off to give the seamless capsules (B) of the present invention containing 25 mg of OMEGA-3-ACID ETHYL ESTERS 90 and 1.25 mg of lapaquistat acetate per capsule.

**Table 1**

| composition ratio | (-) |
|---|---|
| OMEGA-3-ACID ETHYL ESTERS 90 | 1000.00 |
| lapaquistat acetate | 50.00 |
| gelatin (jelly strength 250 g) | 363.56 |
| concentrated glycerin | 42.90 |
| sorbitol | 21.45 |
| yellow dye No. 5 | 0.090 |
| purified water | 998.67 |

### Example 2

According to the composition ratio in the following Table 2, the seamless capsule (B) of the present invention was produced.
Gelatin (jelly strength 261 g) (6372.0 g), concentrated glycerin (750.0 g), sorbitol solution (540.0 g) (solid content: 378.0 g), and yellow dye No. 5 (1.56 g) were dissolved in purified water (22338 g) heated to 52°C, and the mixture was degassed under reduced pressure to give an aqueous shell composition solution.
To OMEGA-3-ACID ETHYL ESTERS 90 (3000 g) was added powdery simvastatin (240 g), and the solid pharmaceutically active ingredient was dispersed by using a dispersion apparatus (manufactured by POLYTRON, KINEMATICA AG) to give a capsule content.
Using an apparatus for seamless capsule production (SPHEREX, manufactured by Freund Corporation), a seamless capsule was prepared from the aqueous shell composition solution and the capsule content. Using MCT oil as a carrier liquid, capsulation was performed at 25 capsules per second.
The obtained capsules were cooled in a refrigerator, and dried in a drier until the water activity reached not more than 0.2. Then the carrier liquid on the capsule surface was wiped off to give the seamless capsules (B) of the present invention containing 25 mg of OMEGA-3-ACID ETHYL ESTERS 90 and 2 mg of simvastatin per capsule.

**Table 2**

| composition ratio | (-) |
|---|---|
| OMEGA-3-ACID ETHYL ESTERS 90 | 1000.00 |
| simvastatin | 80.00 |
| gelatin (jelly strength 261 g) | 360.16 |
| concentrated glycerin | 42.39 |
| sorbitol | 21.37 |
| yellow dye No. 5 | 0.088 |
| purified water | 1271.74 |

### Example 3

According to the composition ratio in the following Table 3, the seamless capsule (B) of the present invention was produced.
Gelatin (jelly strength 261 g) (3186.0 g), concentrated glycerin (375.0 g), sorbitol solution (270.0 g) (solid content: 189.0 g), and yellow dye No. 5 (0.78 g) were dissolved in purified water (11169 g) heated to 52°C, and the mixture was degassed under reduced pressure to give an aqueous shell composition solution.
To OMEGA-3-ACID ETHYL ESTERS 90 (3000 g) was added powdery atorvastatin (240 g), and the solid pharmaceutically active ingredient was dispersed by using a dispersion apparatus (manufactured by POLYTRON, KINEMATICA AG) to give a capsule content.
Using an apparatus for seamless capsule production (SPHEREX, manufactured by Freund Corporation), a seamless capsule was prepared from the aqueous shell composition solution and the capsule content. Using MCT oil as a carrier liquid, capsulation was performed at 25 capsules per second.
The obtained capsules were cooled in a refrigerator, and dried in a drier until the water activity reached not more than 0.2. Then the carrier liquid on the capsule surface was wiped off to give the seamless capsules (B) of the present invention containing 25 mg of OMEGA-3-ACID ETHYL ESTERS 90 and 2 mg of atorvastatin per capsule.

**Table 3**

| composition ratio | (-) |
|---|---|
| OMEGA-3-ACID ETHYL ESTERS 90 | 1000.00 |
| atorvastatin | 80.00 |
| gelatin (jelly strength 261 g) | 360.16 |
| concentrated glycerin | 42.39 |
| sorbitol | 21.37 |
| yellow dye No. 5 | 0.088 |
| purified water | 1271.74 |

### Example 4

According to the composition ratio in the following Table 4, the seamless capsule (A) of the present invention was produced.
Gelatin (jelly strength 250 g) (7517.5 g), concentrated glycerin (902.1 g), sorbitol solution (644.4 g) (solid content: 451.1 g), and yellow dye No. 5 (1.859 g) were dissolved in purified water (20807.8 g) heated to 52°C, and the mixture was degassed under reduced pressure to give a high concentration aqueous shell composition solution.
Powdery lapaquistat acetate (525 g) was added to heated purified water (2000 g), and the mixture was applied to a dispersion apparatus (POLYTRON, manufactured by KINEMATICA AG) to give a dispersion of solid pharmaceutically active ingredient. The solution was added to high concentration aqueous shell composition solution (10000 g), and the mixture was stirred, and degassed under reduced pressure to give an aqueous shell composition solution containing a solid pharmaceutically active ingredient dispersed therein.
To the high concentration aqueous shell composition solution was added heated purified water. The mixture was diluted to a solid content concentration of 25% (2 kg of purified water was added per 10 kg), stirred and degassed under reduced pressure to give an aqueous shell composition solution. As a capsule content, OMEGA-3-ACID ETHYL ESTERS 90 (5616 g) was used. Using an apparatus for seamless capsule production (SPHEREX, manufactured by Freund Corporation), a seamless capsule having a double film structure comprising an inner shell layer formed from the aqueous shell composition solution wherein a solid pharmaceutically active ingredient was dispersed (first layer), and an outer shell layer formed from the aqueous shell composition solution (second layer) was prepared. Using MCT oil as a carrier liquid, capsulation was performed at 20 capsules per second. The obtained capsules were cooled in a refrigerator, and dried in a drier until the water activity reached not more than 0.2. Then the carrier liquid on the capsule surface was wiped off to give the seamless capsule (A) of the present invention containing 20 mg of OMEGA-3-ACID ETHYL ESTERS 90 and 1.25 mg of lapaquistat acetate per capsule.

**[Table 4]**

| composition ratio | (-) |
|---|---|
| OMEGA-3-ACID ETHYL ESTERS 90 | 1000.00 |
| lapaquistat acetate | 50.17 |
| gelatin (jelly strength 250 g) | 725.12 |
| concentrated glycerin | 87.02 |
| sorbitol | 43.51 |
| yellow dye No. 5 | 0.179 |
| purified water | 2216.86 |

### Example 5

According to the composition ratio in the following Table 5, the seamless capsule (A) of the present invention was produced.
Gelatin (jelly strength 261 g) (3186.0 g), concentrated glycerin (375.0 g), sorbitol solution (270.0 g) (solid content: 189 g), and yellow dye No. 5 (0.780 g) were dissolved in purified water (8669.0 g) heated to 52°C, and the mixture was degassed under reduced pressure to give 30% concentration aqueous shell composition solution.
Powdery simvastatin (140 g) was added to heated purified water (333 g), and the mixture was applied to a dispersion apparatus (POLYTRON, manufactured by KINEMATICA AG) to give a dispersion of solid pharmaceutically active ingredient. The solution was added to 30% concentration aqueous shell composition solution (1667 g), and the mixture was stirred, and degassed under reduced pressure to give an aqueous shell composition solution containing a pharmaceutically active ingredient dispersed therein.
To the 30% concentration aqueous shell composition solution was added heated purified water. The mixture was diluted to a solid content concentration of 25% (2 kg of purified water was added per 10 kg), stirred and degassed under reduced pressure to give an aqueous shell composition solution.
As a capsule content, OMEGA-3-ACID ETHYL ESTERS 90 (5000 g) was used.
Using an apparatus for seamless capsule production (SPHEREX, manufactured by Freund Corporation), a seamless capsule having a double film structure comprising an inner shell layer formed from the aqueous shell composition solution wherein a solid pharmaceutically active ingredient was dispersed (first layer), and an outer shell layer formed from the aqueous shell composition solution (second layer) was prepared. Using MCT oil as a carrier liquid, capsulation was performed at 20 capsules per second.
The obtained capsules were cooled in a refrigerator, and dried in a drier until the water activity reached not more than 0.2. Then the carrier liquid on the capsule surface was wiped off to give the seamless capsule (A) of the present invention containing 25 mg of OMEGA-3-ACID ETHYL ESTERS 90 and 2 mg of simvastatin per capsule.

**[Table 5]**

| composition ratio | (-) |
|---|---|
| OMEGA-3-ACID ETHYL ESTERS 90 | 1000.00 |
| simvastatin | 80.00 |
| gelatin (jelly strength 261 g) | 728.64 |
| concentrated glycerin | 85.76 |
| sorbitol | 43.23 |
| yellow dye No. 5 | 0.178 |
| purified water | 2572.67 |

### Example 6

According to the composition ratio in the following Table 6, the seamless capsule (C) of the present invention was produced.
Gelatin (1) (jelly strength 245 g) (3158 g), gelatin (2) (jelly strength 297 g) (1579 g), concentrated glycerin (559.0 g), sorbitol solution (399.2 g) (solid content: 299.4 g), and yellow dye No. (51.17 g) were dissolved in purified water (16610 g) heated to 52°C, and the mixture was degassed under reduced pressure to give an aqueous shell composition solution.
As a capsule content, OMEGA-3-ACID ETHYL ESTERS 90 (11000 g) was used.
Using an apparatus for seamless capsule production (SPHEREX, manufactured by Freund Corporation), a seamless capsule was prepared from the capsule content and the aqueous shell composition solution. Using MCT oil as a carrier liquid, capsulation was performed at 25 capsules per second The obtained capsules were cooled in a refrigerator, and dried in a drier until the water activity reached not more than 0.2. Then the carrier liquid on the capsule surface was wiped off to give a capsule containing a liquid pharmaceutically active ingredient. Erythritol (210 g) and gum arabic (80 g) were dissolved in purified water (800 g), and powdery lapaquistat acetate (50 g), crystalline cellulose (20 g) and talc (40 g) were added. The mixture was dispersed and mixed in a dispersion apparatus (manufactured by IKA) to give a coating solution containing a solid pharmaceutically active ingredient dispersed therein. Using a rotary fluidized-bed granulator (MP-10, manufactured by POWREX CORPORATION), the coating solution containing the solid pharmaceutically active ingredient dispersed therein was sprayed on the capsule containing the liquid pharmaceutically active ingredient, and dried to form a coating, whereby the seamless capsule (C) of the present invention containing 25 mg of OMEGA-3-ACID ETHYL ESTERS 90 and 1.25 mg of lapaquistat acetate per capsule was obtained.

**[Table 6]**

| composition ratio | (-) |
|---|---|
| OMEGA-3-ACID ETHYL ESTERS 90 | 1000.00 |
| gelatin (1)(jelly strength 245 g) | 242.94 |
| gelatin (2) (jelly strength 297 g) | 121.47 |
| concentrated glycerin | 43.00 |
| sorbitol | 21.50 |
| yellow dye No. 5 | 0.090 |
| purified water | 1287.00 |
| lapaquistat acetate | 50.0 |
| erythritol | 210.0 |
| gum arabic powder | 80.0 |
| talc | 40.0 |
| crystalline cellulose | 20.0 |
| purified water | 800.0 |

### Example 7

According to the composition ratio in the following Table 7, the seamless capsule (C) of the present invention was produced.
Gelatin (1) (jelly strength 245 g) (3158 g), gelatin (2) (jelly strength 297 g) (1579 g), concentrated glycerin (559.0 g), sorbitol solution (399.2 g) (solid content: 299.4 g), and yellow dye No. 5 (1.17 g) were dissolved in purified water (16610 g) heated to 52°C, and the mixture was degassed under reduced pressure to give an aqueous shell composition solution. As a capsule content, OMEGA-3-ACID ETHYL ESTERS 90 (11000 g) was used.
Using an apparatus for seamless capsule production (SPHEREX, manufactured by Freund Corporation), a seamless capsule was prepared from the capsule content and the aqueous shell composition solution. Using MCT oil as a carrier liquid, capsulation was performed at 25 capsules per second.
The obtained capsules were cooled in a refrigerator, and dried in a drier until the water activity reached not more than 0.2. Then the carrier liquid on the capsule surface was wiped off to give a capsule containing a liquid pharmaceutically active ingredient.
Erythritol (180 g) and gum arabic (80 g) were dissolved in purified water (800 g), and powdery simvastatin (80 g), crystalline cellulose (20 g) and talc (40 g) were added. The mixture was dispersed and mixed in a dispersion apparatus (manufactured by IKA) to give a coating solution containing a solid pharmaceutically active ingredient dispersed therein.
Using a rotary fluidized-bed granulator (MP-10, manufactured by POWREX CORPORATION), the coating solution containing the solid pharmaceutically active ingredient dispersed therein was sprayed on the capsule containing the liquid pharmaceutically active ingredient, and dried to form a coating, whereby the seamless capsule (C) of the present invention containing 25 mg of OMEGA-3-ACID ETHYL ESTERS 90 and 2 mg of simvastatin per capsule was obtained.

**[Table 7]**

| composition ratio | (-) |
|---|---|
| OMEGA-3-ACID ETHYL ESTERS 90 | 1000.00 |
| gelatin (1)(jelly strength 245 g) | 242.94 |
| gelatin (2)(jelly strength 297 g) | 121.47 |
| concentrated glycerin | 43.00 |
| sorbitol | 21.50 |
| yellow dye No. 5 | 0.090 |
| purified water | 1287.00 |
| simvastatin | 80.0 |
| erythritol | 180.0 |
| gum arabic powder | 80.0 |
| talc | 40.0 |
| crystalline cellulose | 20.0 |
| purified water | 800.0 |

### Example 8

According to the composition ratio in the following Table 8, the seamless capsule (C) of the present invention was produced.
Gelatin (1) (jelly strength 245 g) (3158 g), gelatin (2) (jelly strength 297 g) (1579 g), concentrated glycerin (559.0 g), sorbitol solution (399.2 g) (solid content: 299.4 g), and yellow dye No. 5 (1.17 g) were dissolved in purified water (16610 g) heated to 52°C, and the mixture was degassed under reduced pressure to give an aqueous shell composition solution. As a capsule content, OMEGA-3-ACID ETHYL ESTERS 90 (11000 g) was used.
Using an apparatus for seamless capsule production (SPHEREX, manufactured by Freund Corporation), a seamless capsule was prepared from the capsule content and the aqueous shell composition solution. MCT oil used as a carrier liquid was encapsulated at 25 capsules per second.
The obtained capsules were cooled in a refrigerator, and dried in a drier until the water activity reached not more than 0.2. Then the carrier liquid on the capsule surface was wiped off to give a capsule containing a liquid pharmaceutically active ingredient.
Granulated sugar (210 g) and gum arabic (80 g) were dissolved in purified water (800 g), and powdery lapaquistat acetate (50 g), crystalline cellulose (20 g) and talc (40 g) were added. The mixture was dispersed and mixed in a dispersion apparatus (manufactured by IKA) to give a coating solution containing a solid pharmaceutically active ingredient dispersed therein.
Using a rotary fluidized-bed granulator (MP-10, manufactured by POWREX CORPORATION), the coating solution containing the solid pharmaceutically active ingredient dispersed therein was sprayed on the capsule containing the liquid pharmaceutically active ingredient, and dried to form a coating, whereby the seamless capsule (C) of the present invention containing 25 mg of OMEGA-3-ACID ETHYL ESTERS 90 and 1.25 mg of lapaquistat acetate per capsule was obtained.

**[Table 8]**

| composition ratio | (-) |
|---|---|
| OMEGA-3-ACID ETHYL ESTERS 90 | 1000.00 |
| gelatin (1)(jelly strength 245 g) | 242.94 |
| gelatin (2) (jelly strength 297 g) | 121.47 |
| concentrated glycerin | 43.00 |
| sorbitol | 21.50 |
| yellow dye No. 5 | 0.090 |
| purified water | 1287.00 |
| lapaquistat acetate | 50.0 |
| granulated sugar | 210.0 |
| gum arabic | 80.0 |
| talc | 40.0 |
| crystalline cellulose | 20.0 |
| purified water | 800.0 |

### Example 9

According to the composition ratio in the following Table 9, the seamless capsule (C) of the present invention was produced.
Gelatin (1) (jelly strength 245 g) (3158 g), gelatin (2) (jelly strength 297 g) (1579 g), concentrated glycerin (559.0 g), sorbitol solution (399.2 g) (solid content: 299.4 g), and yellow dye No. 5 (1.17 g) were dissolved in purified water (16610 g) heated to 52°C, and the mixture was degassed under reduced pressure to give an aqueous shell composition solution As a capsule content, OMEGA-3-ACID ETHYL ESTERS 90 (11000 g) was used.
Using an apparatus for seamless capsule production (SPHEREX, manufactured by Freund Corporation), a seamless capsule was prepared from the capsule content and the aqueous shell composition solution. Using MCT oil as a carrier liquid, capsulation was performed at 25 capsules per second.
The obtained capsules were cooled in a refrigerator, and dried in a drier until the water activity reached not more than 0.2. Then the carrier liquid on the capsule surface was wiped off to give a capsule containing a liquid pharmaceutically active ingredient.
Granulated sugar (160 g) and gum arabic (80 g) were dissolved in purified water (800 g), and powdery simvastatin (80 g), crystalline cellulose (20 g), titanium oxide (20 g) and talc (40 g) were added. The mixture was dispersed and mixed in a dispersion apparatus (manufactured by IKA) to give a coating solution containing a solid pharmaceutically active ingredient dispersed therein.
Using a rotary fluidized-bed granulator (MP-10, manufactured by POWREX CORPORATION), a coating solution containing the solid pharmaceutically active ingredient dispersed therein was sprayed on a capsule containing the liquid pharmaceutically active ingredient, and dried to form a coating, whereby the seamless capsule (C) of the present invention containing 25 mg of OMEGA-3-ACID ETHYL ESTERS 90 and 2 mg of simvastatin per capsule was obtained.

**[Table 9]**

| composition ratio | (-) |
|---|---|
| OMEGA-3-ACID ETHYL ESTERS 90 | 1000.00 |
| gelatin (1)(jelly strength 245 g) | 242.94 |
| gelatin (2)(jelly strength 297 g) | 121.47 |
| concentrated glycerin | 43.00 |
| sorbitol | 21.50 |
| yellow dye No. 5 | 0.090 |
| purified water | 1287.00 |
| simvastatin | 80.0 |
| granulated sugar | 160.0 |
| gum arabic powder | 80.0 |
| talc | 40.0 |
| crystalline cellulose | 20.0 |
| titanium oxide | 20.0 |
| purified water | 800.0 |

### Control Example 1 _

According to the formulation shown in Table 10, a preparation (film-coated tablet) was produced.
Lapaquistat acetate (3776 g), lactose hydrate (12270 g) and cornstarch (4500 g) were placed in a fluid bed granulator (FD-S2, manufactured by POWREX CORPORATION), mixed with residual heat, and 5% aqueous hydroxypropylcellulose solution (13510 g) was sprayed to give a granulated powder of lapaquistat acetate. The obtained granulated powder (19810 g) of lapaquistat acetate was applied to a powermill (manufactured by SHOWA KAGAKUKIKAI CO., LTD.) to give a sieved powder. The granulation and sieve were performed twice, and to the obtained sieved powder (38210 g) of lapaquistat acetate were added carmellose calcium (2025 g) and magnesium stearate (270 g) to give a mixed granule. The mixed granule was tableted by a tableting machine (AQUARIUS 36K, manufactured by KIKUSUI SEISAKUSHO LTD.) using a round type (9.5 mm) punch into tablets weighing 300 mg per tablet. Onto the obtained tablets (34500 g) was sprayed a film coating solution [comprising hydroxypropylmethylcellulose (2244 g), polyethylene glycol 6000 (450 g), titanium oxide (300 g) and red ferric oxide (6 g)] by a pan-type coater (Hi-Coater HCF-100N, manufactured by Freund Corporation) to apply a coating (10 mg per tablet), whereby control film-coated tablets containing 50 mg of lapaquistat acetate per tablet was obtained.

**[Table 10]**

| component | content (mg) per tablet |
|---|---|
| lapaquistat acetate | 50.0 |
| lactose hydrate | 164.0 |
| cornstarch | 60.0 |
| Hydroxypropylcellulose aqueous solution | 9.0 |
| carmellose calcium | 15.0 |
| magnesium stearate | 2.0 |
| hydroxypropylmethylcellulose | 7.48 |
| polyethylene glycol 6000 | 1.5 |
| titanium oxide | 1.0 |
| red ferric oxide | 0.02 |

### Control Example 2

Gelatin (1) (jelly strength 245 g) (3158 g), gelatin (2) (jelly strength 297 g) (1579 g), concentrated glycerin (559.0 g), sorbitol solution (399.2 g) (solid content: 299.4 g), and yellow dye No. 5 (1.17 g) were dissolved in purified water (16610 g) heated to 52°C, and the mixture was degassed under reduced pressure to give an aqueous shell composition solution. As a capsule content, OMEGA-3-ACID ETYYL ESTERS 90 (11000 g) was used.
Using an apparatus for seamless capsule production (SPHEREX, manufactured by Freund Corporation), a seamless capsule was prepared from the capsule content and the aqueous shell composition solution. Using MCT oil as a carrier liquid, capsulation was performed at 25 capsules per second.
The obtained capsules were cooled in a refrigerator, and dried in a drier until the water activity reached not more than 0.2. Then the carrier liquid on the capsule surface was wiped off to give a control seamless capsule containing 25 mg of OMEGA-3-ACID ETYYL ESTERS 90 per capsule.

**[Table 11]**

| composition ratio | (-) |
|---|---|
| OMEGA-3-ACID ETHYL ESTERS 90 | 1000.00 |
| gelatin (1) (jelly strength 245 g) | 242.94 |
| gelatin (2) (jelly strength 297 g) | 121.97 |
| concentrated glycerin | 43.00 |
| sorbitol | 21.50 |
| yellow dye No. 5 | 0.090 |
| purified water | 1287.00 |

### Control Example 3

As a preparation containing simvastatin, ZOCOR 80 mg tablet (manufactured by Merck & Co., Inc.) was used.

### Evaluation Example 1

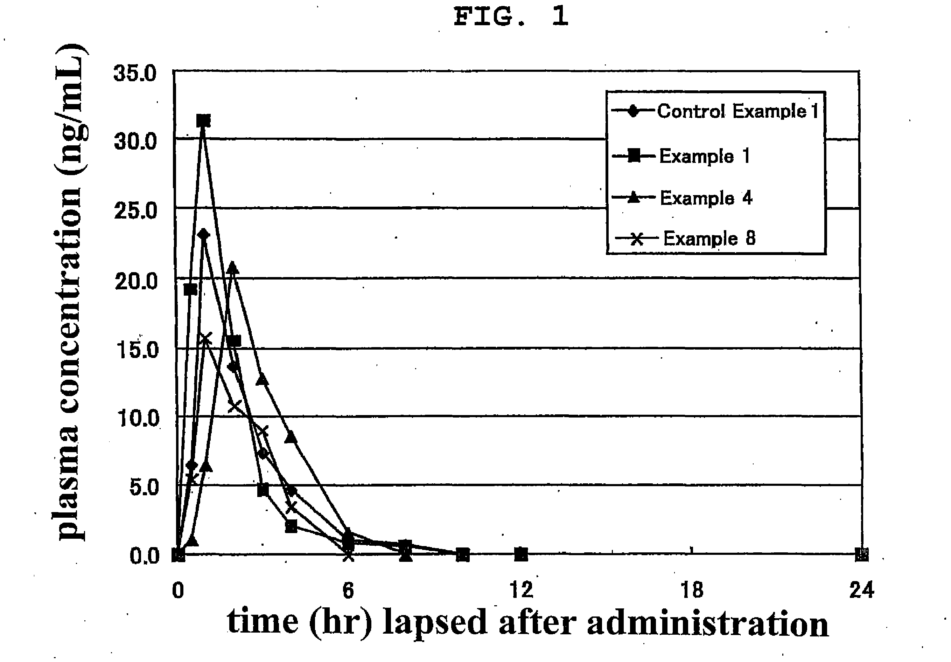

Using the concentration of lapaquistat acetate in plasma as an index, bioabsorbability was evaluated.
The preparations produced in Example 1, Example 4 and Example 8, and the preparation of Control Example 1 were administered by gavage to a dog, blood samples were collected over time and the concentration of lapaquistat acetate in plasma was evaluated.
The preparations of Example 1, Example 4 and Example 8, and the preparation of Control Example 1 were administered to the same dog after a 1 week administration pause period. The dog was fasted from the evening of the previous day of each administration day and, on the administration day, fed after the completion of blood sample collection 6 hr after administration.
A pentagastrin intramuscular administration solution (about 6 µg/kg) was intramuscularly administered 10 min before administration of each preparation and 5 min after administration thereof. The administration method of the preparations was as follows. Purified water (about 10 mL) was given with a disposable syringe immediately before administration of the preparations, the preparations of Example 1, Example 4 and Example 8 were given by one administration unit (40 seamless capsules), one tablet of the preparation of Control Example 1 was administered by gavage, and further, purified water (about 40 mL) was additionally given.
The blood samples were collected before administration and 0.5, 1, 2, 3, 4, 6, 8, 10, 12, 24 and 48 hr (total 12 time points) after administration. The blood samples before administration were collected before administering pentagastrin. The method of collecting blood samples and separating plasma included collecting not less than about 2.5 mL of blood from cephalic vein of forearm with a disposable syringe added with heparin sodium (Novo-Heparin 10,000 units: manufactured by MOCHIDA PHARMACEUTICAL CO., LTD.). The collected blood was centrifuged (4°C, 3,000 rpm, 15 min) to give not less than 0.5 mL of plasma. The obtained plasma was dispensed to a sample tube and cryopreserved in a freezer at - 30°C (MDF-U537D, manufactured by SANYO Electric Biomedical Co., Ltd., temperature set to -30°C, tolerable range -20°C to -40°C). The concentration (ng/mL) of lapaquistat acetate in this cryopreserved plasma was measured by LC/MS/MS test. The results are shown in Fig. 1. The preparations of Example 1, Example 4 and Example 8 showed lapaquistat acetate absorption profile equivalent to that of the preparation of Comparative Example 1. Therefrom it was shown that, even when the seamless capsule of the present invention was used, bioabsorbability of the pharmaceutically active ingredient contained therein was equivalent to that of a single agent.

### Evaluation Example 2

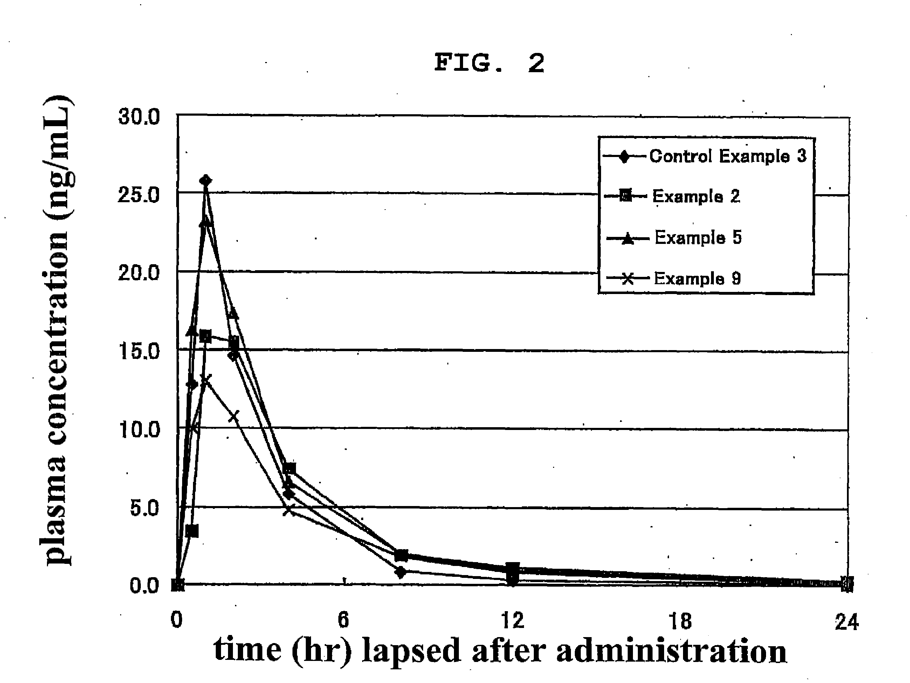

Using the concentration of simvastatin in plasma as an index, bioabsorbability was evaluated.
The preparations produced in Example 2, Example 5 and Example 9, and the preparation of Control Example 3 were administered by gavage to a dog, blood samples were collected over time and the concentration in plasma was evaluated.
The preparations of Example 2, Example 5 and Example 9, and the preparation of Control Example 3 were administered to the same dog after a 1 week administration pause period. The dog was fasted from the evening of the previous day of each administration day and, on the administration day, fed after the completion of blood sample collection 12 hr after administration.
A pentagastrin intramuscular administration solution (about 6 µg/kg) was intramuscularly administered 10 min before administration of each preparation and 5 min after administration thereof.
The administration method of the preparations was as follows. Purified water (about 10 mL) was given with a disposable syringe immediately before administration of the preparations, the preparations of Example 2, Example 5 and Example 9 were given by one administration unit (40 seamless capsules), one tablet of the preparation of Control Example 3 was administered by gavage, and further, purified water (about 40 mL) was additionally given.
The blood samples were collected before administration and 0.5, 1, 2, 4, 8, 12, 24 and 48 hr (total 9 time points) after administration. The blood samples before administration were collected before administering pentagastrin. The method of collecting blood samples and separating plasma included collecting about 2.0 mL of blood from cephalic vein of forearm with a disposable syringe added with heparin sodium (Novo-Heparin 10,000 units: manufactured by MOCHIDA PHARMACEUTICAL CO., LTD.). The collected blood was centrifuged (4°C, 3,000 rpm, 15 min) to give not less than 0.5 mL of plasma. The obtained plasma was dispensed to a sample tube and cryopreserved in a freezer at -30°C (MDF-U537D, manufactured by SANYO Electric Biomedical Co., Ltd., temperature set to -30°C, tolerable range -20°C to -40°C). The concentration (ng/mL) of simvastatin in this cryopreserved plasma was measured by LC/MS/MS test.
The results are shown in Fig. 2. The preparations of Example 2, Example 5 and Example 9 showed simvastatin absorption profile equivalent to that of the preparation of control Example 3. Therefrom it was shown that, even when the seamless capsule of the present invention was used, bioabsorbability of the pharmaceutically active ingredient contained therein was equivalent to that of a single agent.

### Evaluation Example 3

The stability of the preparation produced in Example 2 was evaluated. The preparation of Example 2 was divided into small portions, placed in glass bottles and plugged. They were preserved for 2 months in a system humidity-conditioned at 40°C, 75%RH (relative humidity 75%), and the residual ratio and analogue (decomposed product derived from simvastatin) were measured. The preparation of Control Example 2 was also preserved in the same manner for analogue evaluation.
The amount of simvastatin in the preparation of Example 2 was quantified as follows. The seamless capsules (20 capsules) of Example 2 were collected, placed in a 100 mL measuring flask, and completely disintegrated with purified water (50 mL) added thereto. Thereafter, acetonitrile was added to 100 mL, the mixture was subjected to a filtration treatment using a filter (Acrodisc GF, 25 mm 0.45 µm PVDF, manufactured by Japan Pall Corporation) and the obtained solution (20 µL) was measured.
The content of simvastatin was measured according to HPLC method under the following conditions.

[Residual rate measurement]
measurement wavelength: 249 nm
column: CAPCELL PAK C18 AQ, 5 µm, 4.6 mm i.d.×15 cm (manufactured by Shiseido Co., Ltd.)
mobile phase: acetonitrile/0.01 mol/L phosphate buffer (pH 3.5) mixed solution (3:2)
oven temperature: around 25°C

### [Analogue measurement]

measurement wavelength: 249 nm column: CAPCELL PAK C18 AQ, 5 µm, 4.6 mm i.d.×15 cm (manufactured by Shiseido Co., Ltd.)
mobile phase A: 0.01 mol/L phosphate buffer (pH
3.5)/acetonitrile mixed solution (14:11)
mobile phase B: 0.01 mol/L phosphate buffer (pH 3.5)/acetonitrile mixed solution (1:4) oven temperature: around 25°C gradient program (linear gradient)

**[Table 12]**

| time (min) | mobile phase A (%) | mobile phase B (%) |
|---|---|---|
| 0 | 100 | 0 |
| 0-30 | 100 | 0 |
| 30-80 | 100→0 | 0→100 |
| 80-125 | 0 | 100 |
| 125-126 | 0→100 | 100→0 |
| 126-140 | 100 | 0 |

The analogue was evaluated as follows. The amounts of increase in the analogues after preservation for 2 months of the preparation of Example 2 and the preparation of control Example 2 were calculated, and the amount of increase in the analogue of the preparation of Control Example 2 was subtracted from that of the preparation of Example 2. An analogue with a negative increase was removed from the calculation.
The results are shown in Table 13. The preparation of Example 2 did not show a decrease in the simvastatin content, and the amount of increase in the analogue was below the threshold value of toxicity confirmation as defined in ICH (International Conference on Harmonisation of Technical Requirements for Registration of Pharmaceuticals for Human Use) guidelines. Therefrom it was shown that the seamless capsule of the present invention is superior in stability.

**[Table 13]**

| | after preservation at 40°C, 75%RH for 2 months |
|---|---|
| residual ratio | 99.6% |
| amount of increase | relative retention time (1.7): |
| in analogue derived from simvastatin | 0.04% |
| | relative retention time (2.0): |
| | 0.13% |

### Industrial Applicability

The seamless capsule of the present invention has advantages in that (1) each of the liquid and solid pharmaceutically active ingredients can achieve bioabsorbability equivalent to that of the corresponding single agent; (2) the dissolution property of each of the liquid and solid pharmaceutically active ingredients can be controlled; (3) each active ingredient can be present stably; (4) the preparation is superior in portability and easy administration since it can be downsized; (5) the production costs can be suppressed as compared to conventional combination agents since the production steps can be simplified; and the like.

This application is based on patent application No. 2008-003634 filed in Japan, the contents of which are incorporated in full herein.

## Claims

1. A seamless capsule comprising one or more kinds of liquid pharmaceutically active ingredients and one or more kinds of solid pharmaceutically active ingredients, **wherein the liquid pharmaceutically active ingredient is encapsulated in the form, of a liquid pharmaceutical composition, and the solid pharmaceutically active ingredient is dispersed in the liquid pharmaceutical composition.**

2. The seamless capsule according to claim 1, wherein the liquid pharmaceutically active ingredient is a ω3-fatty acid ethyl ester.

3. The seamless capsule according to any one of claims 1 to 2, wherein the liquid pharmaceutical composition comprises not less than 90% w/w of a ω3-fatty acid ethyl ester.

4. The seamless capsule according to claim 3, wherein the ω3-fatty acid ethyl ester comprises EPA ethyl ester and DHA ethyl ester and the liquid pharmaceutical composition comprises not less than 80% w/w in total of these two components.

5. The seamless capsule according to claim 4, wherein the liquid pharmaceutical composition comprises not less than 40% w/w of EPA ethyl ester and not less than 34% w/w of DHA ethyl ester.

6. The seamless capsule according to claim 5, wherein the liquid pharmaceutical composition is OMEGA-3-ACID ETHYL ESTERS 90 in the European Pharmacopoeia.

7. The seamless capsule according to claim 1, wherein the solid pharmaceutically active ingredient is lapaquistat acetate.

8. The seamless capsule according to claim 1, wherein the solid pharmaceutically active ingredient is statin.

9. The seamless capsule according to claim 8, wherein the solid pharmaceutically active ingredient is atorvastatin.

10. The seamless capsule according to claim 8, wherein the solid pharmaceutically active ingredient is simvastatin.
